# EUROPEAN PATENT SPECIFICATION

(11) **EP 4 326 116 B1**
(45) Date of publication and mention of the grant of the patent: **02.07.2025**
(21) Application number: 22723188.3
(22) Date of filing: 20.04.2022
(51) Int. Cl.: A45B 9/00, A61B 5/00, A61B 5/0205, A45B 3/00, A45B 3/04, A61B 5/021, A61B 5/024, A61B 5/11, A61B 5/145, A61B 5/22

(54) **SET FOR MOTOR ACTIVITIES AND SYSTEM FOR ACQUISITION AND PROCESSING OF DATA DETECTED BY THE SET FOR MOTOR ACTIVITIES AND CORRESPONDING OPERATING METHOD**
SET FÜR MOTORAKTIVITÄTEN UND SYSTEM ZUR ERFASSUNG UND VERARBEITUNG VON DURCH DAS SET ERKANNTEN DATEN FÜR MOTORAKTIVITÄTEN UND ZUGEHÖRIGES BETRIEBSVERFAHREN
ENSEMBLE POUR ACTIVITÉS MOTRICES ET SYSTÈME D'ACQUISITION ET DE TRAITEMENT DES DONNÉES DÉTECTÉES PAR L'ENSEMBLE POUR ACTIVITÉS MOTRICES ET PROCÉDÉ DE FONCTIONNEMENT CORRESPONDANT

(30) Priority: 20.04.2021 IT 202100010013
(43) Date of publication of application: 28.02.2024
(73) Proprietor: Pimpinato, Monica, 36061 Bassano del Grappa (VI) (IT)
(72) Inventor: Pimpinato, Monica, 36061 Bassano del Grappa (VI) (IT)
(74) Representative: Casadei, Barbara
(86) International application number: PCT/IB2022/053684
(87) International publication number: WO 2022/224157

(56) References cited:
- EP-A1- 1 908 499
- CN-A- 109 619 780

## Description

### Technical field

The present invention relates to a set for motor activities and a system for acquisition and processing of data detected by the set for motor activities and a corresponding operating method.

### Background art

The term set for motor activities means a pair of sticks.

The sticks referred to are used, for example, in Nordic walking, trail running or winter skiing in all their types.

The sticks may be used, for example, in assisted walking as an aid and/or support for rehabilitation activities.

In order to be able to keep track of the method of use of the sticks during a motor session, whether it is of an amateur or competitive or professional type, "technological" sticks have been developed over recent years, that is to say, provided with integrated electronics to allow a motor-kinematic analysis focussed on the movement of each stick and, consequently, on the upper part of the user's body.

In effect, there are prior art solutions of sticks provided with one or more sensors, each dedicated for detecting a respective physical characteristic, and an electronic module configured to manage the data detected by the sensors.

Although there are currently prior art solutions which make it possible to detect data useful for analysing a motor session of a user, with the aim of detecting any corrective actions or to observe actual progress, there has been a difficulty in associating the data acquired from the electronic module of a stick with the data acquired of the electronic module of the other stick, resulting in considerable interpretation limitations on the method of use of the pair of sticks during the motor session.

An example of a prior art solution is described in patent document EP1908499 A1 wherein the sticks, provided with sensors, communicate with each other via radio in order to synchronise the measurements acquired by the sticks and to transmit the data to a processing unit.

The synchronisation of the sticks by means of radio communication is not free of drawbacks, since it cannot guarantee the correct association of the data acquired by the pair of sticks.

In fact, it should be noted that a delay in communication between the two sticks during the training session inevitably results in the data transmitted not being correctly associated, resulting in an incorrect interpretation of the means of use of the pair of sticks.

The need has therefore been felt of making a set for motor activities which comprises a first stick for walking and a second stick for walking.

### Disclosure of the invention

The set for motor activities of the present invention is defined in claim 1. The first stick and the second stick each comprise one or more sensors for detecting respective physical characteristics, an electronic module which comprises a respective electronic unit for detecting and recording data acquired by the sensor.

The electronic units of the first stick and of the second stick are configured for communicating with each other using a wireless data transmission network.

Each electronic unit is configured to receive an activation command for starting recording of the data acquired and a deactivation command for ending recording.

Each electronic unit is configured to receive a timestamp at the start of data recording.

Advantageously, this allows the pair of sticks to record the data detected by the sensors in a perfectly synchronous fashion, so as to be able to associate the data acquired by the two electronic modules without errors in a subsequent step of processing the data recorded.

The invention also relates to a system for acquisition and processing of data detected by the set for motor activities according to the invention and a corresponding operating method.

### Brief description of drawings

Further features and advantages of this invention are more apparent in the detailed description below, with reference to a preferred, non-limiting, embodiment of a set for motor activities and relative data processing system as illustrated in the accompanying drawings, in which:

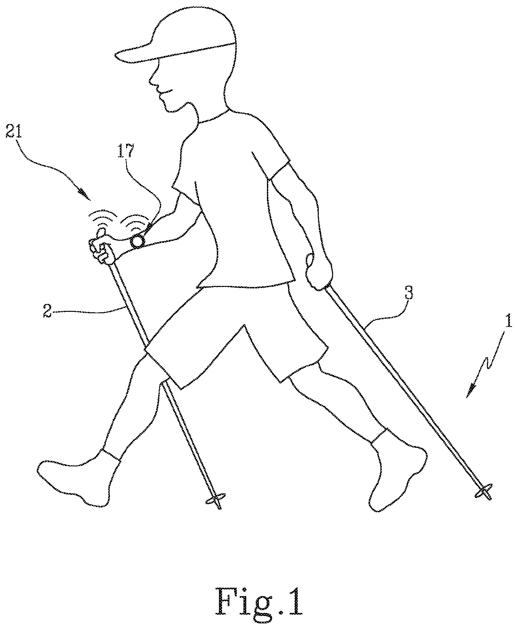

- Figure 1 illustrates a set for motor activities according to the invention;

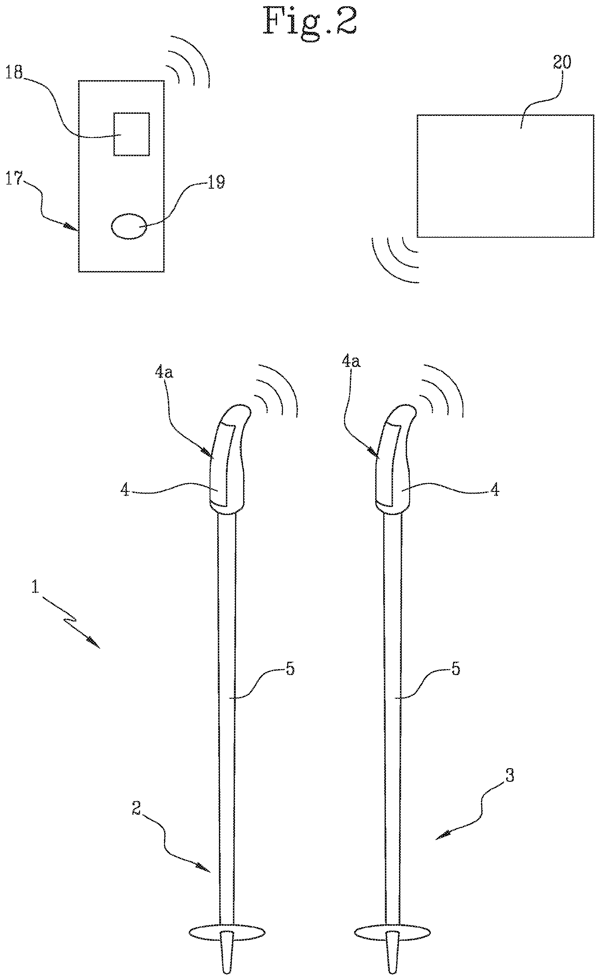

- Figure 2 illustrates a diagram of a system for acquisition and processing of data detected by the set for motor activities according to the invention;
- Figure 3 illustrates a block diagram of the electronic modules of the set for motor activities according to the invention.

### Detailed description of preferred embodiments of the invention

With reference to the accompanying drawings, the numeral 1 denotes a set for motor activities according to the invention.

The set for motor activities 1 comprises a first stick 2 for walking and a second stick 3 for walking.

The first stick 2 and the second stick 3 each comprise a respective handgrip 4, having a gripping zone 4a, and a respective stem 5, extending from the handgrip 4.

The first stick 2 and the second stick 3 each comprise a respective electronic module 6.

With reference to the first stick 2 and to the second stick 3, the electronic module 6 is contained in a case inserted inside a relative housing made in the handgrip 4.

The electronic module 6 has an activating and deactivating element 7, preferably in the form of a pushbutton which can be operated by the user. The electronic module 6 comprises one or more sensors 8 for detecting respective physical characteristics.

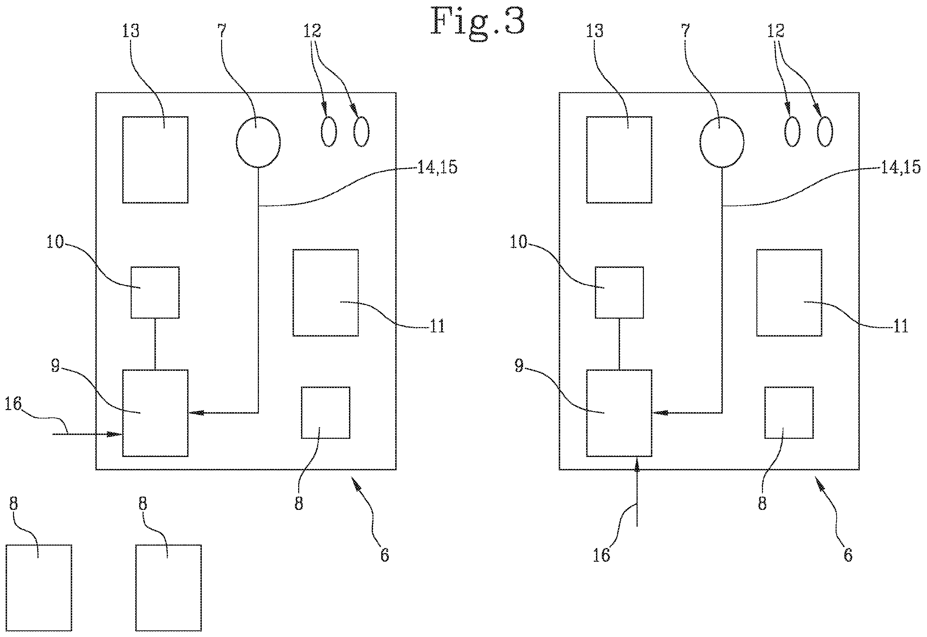

The sensor 8 may be physically connected to the electronic module 6 or be connected by a wireless data connection, as schematically illustrated schematically in Figure 3.

For example, a sensor used may be a sensor detecting the presence of a hand of the user, preferably in the form of a pushbutton.

Or a sensor for detecting the gripping force of the hand, preferably a sensor of the piezoelectric and/or electro-resistive and/or barometric layer type.

Further sensors used can be a motion sensor, an accelerometer, a gyroscope, a force sensor, a dynamometer, a load cell, a magnetometer, a speed sensor, a geolocation module, a temperature detector, a moisture detector, an inclinometer, an atmospheric pressure meter.

The sensors referred to may also be understood as physiological sensors such as, for example, a blood pressure and/or body temperature and/or heart rate and/or blood oxygenation saturation detector.

The electronic module 6 comprises an electronic unit 9 for detecting and recording data acquired by the sensor 8.

Each electronic unit 9 has a data recording frequency of from 20 to 300 Hertz, included.

Each electronic unit 9 is configured to receive an activation command 14 for starting recording of the data acquired and a deactivation command 15 for ending recording.

Each electronic unit 9 is configured for storing the data acquired by the sensor 8 in a respective memory section 10 configured for transmitting the data to the outside.

Alternatively, at least one between the two electronic units 9 of the first stick 2 and of the second stick 3 is configured to send the data acquired to the memory section 10 of the other electronic unit 9.

In this way, it is possible to extract the data acquired by the first stick 2 and by the second stick 3 accessing a single memory section 10.

Preferably, the memory section 10 is of the flash memory type.

The memory section 10 is accessible from the outside by means of a wireless data transmission network and/or by a cable connection.

The electronic module 6 comprises a power unit 11, preferably of the rechargeable type, for powering at least the electronic unit 9 and, optionally, the one or more sensors 8 if physically connected to the electronic module 6.

The electronic module 6 has one or more optical indicators 12 configured to signal one or more operating states of the electronic module 6, as described below.

Preferably, the electronic module 6 has two optical indicators 12, each having a different light source colour, for example according to an optical indicator 12 having a red light source and an optical indicator 12 having a blue light source.

With reference to the power unit 11, the optical indicator 12 is configured to be activated during the charging operation of the power unit 11 and to deactivate after charging has been completed, in a preferred manner, the optical indicator 12 activates and deactivates a red light source.

The electronic module 6 comprises an emitter 13 of a detection signal 16, preferably in the form of radio waves.

Each electronic unit 9 of an electronic module 6 is configured to receive the detection signal 16 emitted by the emitter 13 of the other electronic module 6.

This means that the electronic units 9 of the first stick 2 and of the second stick 3 are configured for exchanging data by means of wireless data transmission network, in particular by radio waves.

According to an aspect of the invention, each electronic unit is configured to receive a timestamp at the start of data recording.

With reference to a set for motor activities 1, one of either of the two electronic units 9 of the first stick 2 and of the second stick 3, is called the reference, or main, electronic unit 9a, and the other is known as the dependent, or secondary, electronic unit 9b.

The reference electronic unit 9a is configured to send a timestamp to the dependent electronic unit 9b.

This makes it possible to simultaneously start the recording of the data acquired by the reference electronic unit 9a and by the dependent electronic unit 9b during a motor session and allows the subsequent superposing of data so as to be able to perform comparative analyses of the movements of the sticks in relation to each other.

The reference electronic unit 9a is configured to send to the dependent electronic unit 9b a timestamp according to a predetermined frequency.

In use, the user activates the electronic module 6 of the first stick 2 and of the second stick 3 by means of the activating and deactivating element 7.

As confirmation of the activation of the electronic module 6, the optical indicator 12 activates by flashing at a first frequency, preferably using the red light source.

The activation of the electronic module 6 of the first stick 2 and of the electronic module 6 of the second stick 3 corresponds with the activation of the emitter 13 of the detection signal 16, preferably in the form of radio waves.

In order to associate the electronic unit 9 of the electronic module 6 of the first stick 2, that is to say, the reference electronic unit 9a, with the electronic unit 9 of the electronic module 6 of the second stick 3, that is to say, the dependent electronic unit 9b, the user acts on the activating and deactivating element 7 of the electronic module 6 of the first stick 2 for at least one second.

In this way, the dependent electronic unit 9b detects the presence of the other reference electronic unit 9a upon receiving the detection signal 16, linking with it.

The association of the electronic module 6 of the first stick 2 and of the electronic module 6 of the second stick 3 is confirmed by the activation of the optical indicator 12, in particular by the synchronous flashing of both light sources, red and blue, at the first frequency.

If the electronic module 6 of the dependent electronic unit 9b is deactivated or outside the range of receiving the detection signal 16 emitted by the dependent electronic unit 9b, the dependent electronic unit 9b associates with the reference electronic unit 9a.

In order to signal this disassociation condition to the user, the optical indicator 12 is configured to activate the light sources, red and blue, in an asynchronous manner.

At this point, in order to start the recording of the data of a motor session, the user presses the element 7 for activating and deactivating the electronic module 6 of the first stick 2 and of the electronic module 6 of the second stick 3 for a time greater than 1 second, in particular 3 seconds.

As confirmation of the activation of the data recording, the optical indicator 12 activates by flashing at a second frequency, different from the first frequency, in particular greater than the first frequency.

In particular, the synchronous flashing of both the light sources, the red and the blue, is activated at the second frequency.

The reference electronic unit 9a of the two electronic modules 6, after receiving the recording activation command 14, sends to the dependent electronic unit 9b the timestamp at the start of recording.

In this way, the recording of the data of the first stick 2 is synchronised with the recording of the data of the second stick 3.

According to a variant embodiment, the electronic units 9 of the first stick 2 and of the second stick 3 are configured to receive the timestamp at the start of recording from an external electronic device.

In this way, the recording of the data of the first stick 2 is also synchronised with the recording of the data of the second stick 3.

Moreover, in use, in order to align with a predetermined frequency the data acquired from the electronic units 9, the reference electronic unit 9a of the two electronic modules 6 sends the timestamp to the dependent electronic unit 9b.

The data of the reference electronic unit 9a and the data of the dependent electronic unit 9b are stored in the respective memory sections 10.

In order to disable the recording, the user presses the activating and deactivation element 7 of the electronic module 6 of the first stick 2 and of the electronic module 6 of the second stick 3 for a time greater than 1 second, in particular 3 seconds.

In this mode, the electronic units 9 receive the deactivation command 15.

As confirmation of the deactivation of the data recording, the optical indicator 12 flashes again at the first frequency.

In order to switch off the electronic modules 6 it is necessary to act again on the activation and deactivation element 7 for a time greater than 1 second, in particular 5 seconds.

As confirmation of deactivation of the electronic modules 6, the light source of the optical indicator 12 is off.

At this point, the data recorded in the memory sections 10 of the electronic unit 9 of the first stick 2 and of the second stick 3 are available to be extrapolated.

The set for motor activities 1 comprising the two electronic modules 6 of the first stick 2 and of the second stick 3 may already in itself constitute an autonomous system for data acquisition of each stick and for storing them inside the respective flash-memory for a subsequent analysis.

The invention also relates to a data acquisition and processing system 21 which comprises at least one set for motor activities 1 as described above and an electronic data processing and acquisition device 17 provided with an application 18 configured for communicating with the reference electronic unit 9a and with the dependent electronic unit 9b using a wireless data transmission network.

The electronic data processing and acquisition device 17 is configured for accessing the Internet.

The electronic data processing and acquisition device 17 may be a smartwatch, a smartphone, a PC, a tablet, or the like.

Preferably, the wireless data transmission network is performed by a radio transmission system.

The application 18 is configured to send local date and time to the electronic units 9 of the first stick and of the second stick 3.

The local date and time of the application 18 is intended to define the timestamp of the reference electronic unit 9a and of the dependent electronic unit 9b.

The electronic device 17 is equipped with a geolocation system 19 and the application 18 is configured to associate the geographic data of the geolocation system 19 with the data recorded in the memory sections 10 of the electronic units 9.

Advantageously, this makes it possible to add to the data recorded in the electronic modules 6 the distance travelled, speed, path followed, and the like.

The application 18 is configured to send the activation command 14 for starting data recording and the deactivation command 15 at the end of data recording to the electronic units 9.

In use, the user activates the electronic module 6 of the first stick 2 and of the second stick 3 by means of the activating and deactivating element 7.

The user acts on the element 7 for activating and deactivating the electronic module 6 of the first stick 2 for at least one second to associate the electronic unit 9 of the electronic module 6 of the first stick 2, that is to say, the reference electronic unit 9a, with the electronic unit 9 of the electronic module 6 of the second stick 3, that is to say, the dependent electronic unit 9b.

Once the electronic unit 9 of the electronic module 6 of the first stick 2, that is to say, the reference electronic unit 9a, is associated with the electronic unit 9 of the electronic module 6 of the second stick 3, that is to say, the dependent electronic unit 9b, the user can act by means of the application 18 to send the activation command 14 for starting data recording and the deactivation command 15 for ending data recording to the electronic units 9.

Advantageously, the use of the application 18 makes it possible to synchronise the electronic units 9 of the first stick 2 and of the second stick 3 at the local date and time, and therefore at a unique timestamp between the electronic modules 6 and the application 18.

The application 18 is configured to receive the data recorded in the memory sections 10 of the electronic units 9 of the first stick 2 and of the second stick 3 and to make them available to a user.

The electronic data processing and acquisition device 17 has means for displaying the data received from the application 18.

The application 18 is configured to send and store the data recorded in the memory sections 10 in a web platform 20.

The application 18 is configured for deleting the data recorded in the memory sections 10 of the electronic units 9.

The application 18 is configured for managing the memory section 10.

The application 18 is configured to check the state of charge of the power unit 11.

The application 18 is configured for updating the firmware of the electronic module 6.

The application 18 is configured to name the electronic module 6 of the first stick 2 and of the second stick 3.

The application 18 is configured to make available to the user one or more reports of a motor session in a reduced and compact form, the report is preferably in a pdf format.

With reference to the web platform 20, it has a personal section accessible through a personal account.

The web platform 20 is configured to receive and process the data recorded by the electronic modules 6 of the first stick 2 and of the second stick 3 from raw data in binary format into data in numerical and graphical format.

The web platform 20 is accessible by a user by means of any electronic device configured to connect to the Internet.

The web platform 20 is configured to receive and process further data entered from the outside.

For example, this data may be voluntary information useful for the characterisation of the user.

In this way it is possible to provide data recorded by the electronic modules 6 and/or further data entered from the outside to perform analyses and provide statistics on the data associated with respective motor sessions.

The web platform 20 is configured to make available to a user the data processed in numerical and/or graphic format associated with a motor session and is configured to extrapolate one or more reports of a motor session derived from the analyses and statistics of the processed data.

The set for motor activities 1 according to the invention makes it possible to process the data recorded by the electronic modules 6 synchronised with each other to obtain information on particular characteristics of the movement and absolute motor behaviour of the upper part of the body in the assisted walking motor activity.

Moreover, the possibility of superposing the data recorded by the electronic modules 6 synchronised with each other allows levels of movement performance to be highlighted with respect to the expected aims and, on the contrary, problems and critical issues from the motor point of view which are encountered in subjects with failings in terms of motor capacity and coordination.

This feature makes the system according to the invention not only a tool for analysis and assessment of a motor-sports nature but also and above all an effective diagnostic tool in the motor-rehabilitation and monitoring sector for the achievement of motor activities aims necessary for persons with motor rehabilitation requirements and to support treatments for individuals with metabolic and heart-circulatory diseases.

## Claims

1. A set for motor activities comprising a first stick (2) for walking and a second stick (3) for walking;
the first stick (2) for walking and the second stick (3) for walking each comprise one or more sensors (8) for detecting respective physical characteristics, an electronic module (6) comprising a respective electronic unit (9) for detecting and recording data acquired by the sensor (8);
the electronic units (9) of the first stick (2) and of the second stick (3) are configured for communicating with each other using a wireless data transmission network;
each electronic unit (9) is configured to receive an activation command (14) for starting recording of the data acquired and a deactivation command (15) for ending recording;
**characterised in that**
at least one electronic unit (9) is configured to receive a timestamp;
one of either of the two electronic units (9) of the first stick (2) and of the second stick (3), called the reference electronic unit (9a), is configured to send to the other electronic unit (9), called the dependent electronic unit (9b), a timestamp.

2. The set for motor activities according to the preceding claim, **characterised in that** each electronic unit (9) is configured for storing the data acquired in a respective memory section (10) accessible from the outside.

3. The set for motor activities according to any one of the preceding claims, **characterised in that** at least one of between the two electronic units (9) of the first stick (2) and of the second stick (3) is configured to send the data acquired to the memory section (10) of the other electronic unit (9).

4. The set for motor activities according to any one of the previous claims, **characterised in that** the electronic unit (9) of the first stick (2) is configured to detect the electronic unit (9) of the second stick (3), and vice versa, by receiving a detection signal (16), preferably a radio signal.

5. The set for motor activities according to any one of the previous claims, **characterised in that** it comprises one or more optical indicators (12) configured to pass from at least a first activation condition to a second activation condition, different from the first activation condition, in relation to an association status and to a disassociation status of the reference electronic unit (9a) with the dependent electronic unit (9b).

6. A data acquisition and processing system comprising at least one set for motor activity (1) according to any one of claims 1 to 5, comprising an electronic data processing and acquisition device (17) equipped with an application (18) configured for communicating with the electronic units (9) of the first stick (2) and of the second stick (3) using a wireless data transmission network; the application (18) is configured to receive the data recorded in the memory sections (10) of the electronic units (9) of the first stick (2) and of the second stick (3) and to make them available to a user; in particular, the application (18) is configured to prepare a report summarising the data detected in the memory sections (10) of the electronic units (9);
**characterised in that**
the application (18) is configured to send local date and time to the electronic units (9); the local date and time of the application (18) is intended to define the timestamp of the reference electronic unit (9a) and of the dependent electronic unit (9b).

7. The system according to claim 6, **characterised in that** the application (18) is configured to send the activation command (14) for starting data recording and the deactivation command (15) at the end of data recording to the electronic units (9).

8. The system according to any one of claims 6 or 7, **characterised in that** the application (18) is configured for deleting the data recorded in the memory sections (10) of the electronic units (9).

9. The system according to any one of claims 6 to 8, **characterised in that** the electronic device (17) is equipped with a geolocation system (19) and the application (18) is configured to associate the geographic data of the geolocation system (19) with the data recorded in the memory sections (10) of the electronic units (9).

10. The system according to any one of claims 6 to 9, **characterised in that** the application (18) is configured to send the data recorded in the memory sections (10) to a web platform (20).

11. The system according to claim 10, **characterised in that** the web platform (20) is configured to receive data from the outside as well as from the application (18).

12. The system according to claim 10 or 11, **characterised in that** the web platform (20) is configured to process the data received from the application (18) and to make available to a user the data processed in numerical and/or graphic format associated with a motor session and is configured to extrapolate one or more reports of a motor session derived from the analyses and statistics of the processed data.

13. A method for the operation of a set for motor activities (1) according to any one of claims 1 to 5, comprising a step of activating recording of the data acquired,
**characterised in that**
this step is associated with a step of sending a timestamp to at least one of the electronic units (9) of the first stick (2) and of the second stick (3);
after the step of activating the recording, a step of sending a timestamp to at least one of the electronic units (9) of the first stick (2) and of the second stick (3) according to a predetermined transmission frequency.

14. The method according to claim 13, **characterised in that** it stores the data acquired by the sensors (8) associated with a relative timestamp.

## Patentansprüche

1. Set für motorische Aktivitäten, umfassend einen ersten Stock (2) zum Gehen und einen zweiten Stock (3) zum Gehen;
wobei der erste Stock (2) zum Gehen und der zweite Stock (3) zum Gehen jeweils einen oder mehrere Sensoren (8) zum Erkennen jeweiliger körperlicher Eigenschaften und ein Elektronikmodul (6), umfassend eine jeweilige Elektronikeinheit (9) zum Erkennen und Aufzeichnen von vom Sensor (8) erfassten Daten, umfassen;
wobei die Elektronikeinheiten (9) des ersten Stocks (2) und des zweiten Stocks (3) ausgelegt sind, um unter Nutzung eines drahtlosen Datenübertragungsnetzwerks miteinander zu kommunizieren;
wobei eine jede Elektronikeinheit (9) ausgelegt ist, um einen Aktivierungsbefehl (14) zum Starten der Aufzeichnung der erfassten Daten und einen Deaktivierungsbefehl (15) zum Beenden der Aufzeichnung zu empfangen;
**dadurch gekennzeichnet, dass**
mindestens eine Elektronikeinheit (9) ausgelegt ist, um einen Zeitstempel zu empfangen;
eine der beiden Elektronikeinheiten (9) des ersten Stocks (2) und des zweiten Stocks (3), die als Referenzelektronikeinheit (9a) bezeichnet wird, ausgelegt ist, um der anderen Elektronikeinheit (9), die als abhängige Elektronikeinheit (9b) bezeichnet wird, einen Zeitstempel zu senden.

2. Set für motorische Aktivitäten nach dem vorhergehenden Anspruch, **dadurch gekennzeichnet, dass** eine jede Elektronikeinheit (9) ausgelegt ist, um die erfassten Daten in einem jeweiligen Speicherabschnitt (10), der von außen zugänglich ist, zu speichern.

3. Set für motorische Aktivitäten nach einem der vorhergehenden Ansprüche, **dadurch gekennzeichnet, dass** mindestens eine der beiden Elektronikeinheiten (9) des ersten Stocks (2) und des zweiten Stocks (3) ausgelegt ist, um die erfassten Daten an den Speicherabschnitt (10) der anderen Elektronikeinheit (9) zu senden.

4. Set für motorische Aktivitäten nach einem der vorhergehenden Ansprüche, **dadurch gekennzeichnet, dass** die Elektronikeinheit (9) des ersten Stocks (2) ausgelegt ist, um durch Empfangen eines Erkennungssignals (16), vorzugsweise eines Funksignals, die Elektronikeinheit (9) des zweiten Stocks (3) zu erkennen und umgekehrt.

5. Set für motorische Aktivitäten nach einem der vorhergehenden Ansprüche, **dadurch gekennzeichnet, dass** es eine oder mehrere optische Anzeigen (12) umfasst, die ausgelegt sind, um in Bezug auf eine Verknüpfungszustand und einen Verknüpfungsaufhebungszustand der Referenzelektronikeinheit (9a) mit der abhängigen Elektronikeinheit (9b) von mindestens einem ersten Aktivierungszustand in einen zweiten Aktivierungszustand zu wechseln, der sich vom ersten Aktivierungszustand unterscheidet.

6. Datenerfassungs- und -verarbeitungssystem, umfassend mindestens ein Set für motorische Aktivitäten (1) nach einem der Ansprüche 1 bis 5, umfassend eine elektronische Datenverarbeitungs- und -erfassungsvorrichtung (17), die mit einer Anwendung (18) ausgestattet ist, die ausgelegt ist, um mit den Elektronikeinheiten (9) des ersten Stocks (2) und des zweiten Stocks (3) unter Nutzung eines drahtlosen Datenübertragungsnetzwerks zu kommunizieren, wobei die Anwendung (18) ausgelegt ist, um die in den Speicherabschnitten (10) der Elektronikeinheiten (9) des ersten Stocks (2) und des zweiten Stocks (3) aufgezeichneten Daten zu empfangen und diese einem Nutzer zur Verfügung zu stellen, wobei die Anwendung (18) insbesondere ausgelegt ist, um einen Bericht zu erstellen, der die in den Speicherabschnitten (10) der Elektronikeinheiten (9) erkannten Daten zusammenfasst;
**dadurch gekennzeichnet, dass**
die Anwendung (18) ausgelegt ist, um das lokale Datum und die lokale Zeit an die Elektronikeinheiten (9) zu senden, wobei das lokale Datum und die lokale Zeit der Anwendung (18) dazu bestimmt sind, den Zeitstempel der Referenzelektronikeinheit (9a) und der abhängigen Elektronikeinheit (9b) zu definieren.

7. System nach Anspruch 6, **dadurch gekennzeichnet, dass** die Anwendung (18) ausgelegt ist, um den Aktivierungsbefehl (14) zum Starten der Datenaufzeichnung und den Deaktivierungsbefehl (15) am Ende der Datenaufzeichnung an die Elektronikeinheiten (9) zu senden.

8. System nach einem der Ansprüche 6 oder 7, **dadurch gekennzeichnet, dass** die Anwendung (18) ausgelegt ist, um die in den Speicherabschnitten (10) der Elektronikeinheiten (9) aufgezeichneten Daten zu löschen.

9. System nach einem der Ansprüche 6 bis 8, **dadurch gekennzeichnet, dass** die elektronische Vorrichtung (17) mit einem Geolokalisierungssystem (19) ausgestattet ist und die Anwendung (18) ausgelegt ist, um die geografischen Daten des Geolokalisierungssystems (19) mit den in den Speicherabschnitten (10) der Elektronikeinheiten (9) aufgezeichneten Daten zu verknüpfen.

10. System nach einem der Ansprüche 6 bis 9, **dadurch gekennzeichnet, dass** die Anwendung (18) ausgelegt ist, um die in den Speicherabschnitten (10) aufgezeichneten Daten an eine Webplattform (20) zu senden.

11. System nach Anspruch 10, **dadurch gekennzeichnet, dass** die Webplattform (20) ausgelegt ist, um Daten von außen sowie von der Anwendung (18) zu empfangen.

12. System nach Anspruch 10 oder 11, **dadurch gekennzeichnet, dass** die Webplattform (20) ausgelegt ist, um die von der Anwendung (18) empfangenen Daten zu verarbeiten und einem Nutzer die verarbeiteten Daten in einem Zahlen- und/oder grafischen Format, verknüpft mit einer motorischen Sitzung, zur Verfügung zu stellen, und ausgelegt ist, um einen oder mehrere Berichte einer motorischen Sitzung abgeleitet von den Analysen und Statistiken der verarbeiteten Daten zu extrahieren.

13. Verfahren für den Betrieb eines Sets für motorische Aktivitäten (1) nach einem der Ansprüche 1 bis 5, umfassend einen Schritt zum Aktivieren der Aufzeichnung der erfassten Daten,
**dadurch gekennzeichnet, dass**
der Schritt mit einem Schritt zum Senden eines Zeitstempels an mindestens eine der Elektronikeinheiten (9) des ersten Stocks (2) und des zweiten Stocks (3) verknüpft ist;
nach dem Schritt zum Aktivieren der Aufzeichnung ein Schritt zum Senden eines Zeitstempels an mindestens eine der Elektronikeinheiten (9) des ersten Stocks (2) und des zweiten Stocks (3) nach einer vorbestimmten Übertragungsfrequenz.

14. Verfahren nach Anspruch 13, **dadurch gekennzeichnet, dass** es die von den Sensoren (8) erfassten Daten verknüpft mit einem relativen Zeitstempel speichert.

## Revendications

1. Ensemble pour activités motrices, comprenant un premier bâton (2) pour marcher et un second bâton (3) pour marcher ;
le premier bâton (2) pour marcher et le second bâton (3) pour marcher comprennent chacun un ou plusieurs capteurs (8) pour détecter des caractéristiques physiques respectives, un module électronique (6) comprenant une unité électronique respective (9) pour détecter et enregistrer des données acquises par le capteur (8) ; les unités électroniques (9) du premier bâton (2) et du second bâton (3) sont configurées pour communiquer entre elles à l'aide d'un réseau de transmission de données sans fil ;
chaque unité électronique (9) est configurée pour recevoir une commande d'activation (14) pour commencer l'enregistrement des données acquises et une commande de désactivation (15) pour terminer l'enregistrement ; **caractérisé en ce que**
au moins une unité électronique (9) est configurée pour recevoir un horodatage ;
l'une des deux unités électroniques (9) du premier bâton (2) et du second bâton (3), appelée unité électronique de référence (9a), est configurée pour envoyer à l'autre unité électronique (9), appelée unité électronique dépendante (9b), un horodatage.

2. Ensemble pour activités motrices selon la revendication précédente, **caractérisé en ce que** chaque unité électronique (9) est configurée pour stocker les données acquises dans une section de mémoire respective (10) accessible de l'extérieur.

3. Ensemble pour activités motrices selon l'une quelconque des revendications précédentes, **caractérisé en ce qu'**au moins l'une des deux unités électroniques (9) du premier bâton (2) et du second bâton (3) est configurée pour envoyer les données acquises à la section de mémoire (10) de l'autre unité électronique (9).

4. Ensemble pour activités motrices selon l'une quelconque des revendications précédentes, **caractérisé en ce que** l'unité électronique (9) du premier bâton (2) est configurée pour détecter l'unité électronique (9) du second bâton (3), et vice versa, en recevant un signal de détection (16), de préférence un signal radio.

5. Ensemble pour activités motrices selon l'une quelconque des revendications précédentes, **caractérisé en ce qu'**il comprend un ou plusieurs indicateurs optiques (12) configurés pour passer d'au moins une première condition d'activation à une seconde condition d'activation, différente de la première condition d'activation, par rapport à un état d'association et à un état de dissociation de l'unité électronique de référence (9a) avec l'unité électronique dépendante (9b) .

6. Système d'acquisition et de traitement des données, comprenant au moins un ensemble d'activité motrice (1) selon l'une quelconque des revendications 1 à 5, comprenant un dispositif électronique de traitement et d'acquisition de données (17) équipé d'une application (18) configurée pour communiquer avec les unités électroniques (9) du premier bâton (2) et du second bâton (3) en utilisant un réseau de transmission de données sans fil ; l'application (18) est configurée pour recevoir les données enregistrées dans les sections de mémoire (10) des unités électroniques (9) du premier bâton (2) et du second bâton (3) et pour les mettre à la disposition d'un utilisateur ; en particulier, l'application (18) est configurée pour préparer un rapport résumant les données détectées dans les sections de mémoire (10) des unités électroniques (9) ;
**caractérisé en ce que**
l'application (18) est configurée pour envoyer une date et une heure locales aux unités électroniques (9) ; la date et l'heure locales de l'application (18) sont destinées à définir l'horodatage de l'unité électronique de référence (9a) et de l'unité électronique dépendante (9b).

7. Système selon la revendication 6, **caractérisé en ce que** l'application (18) est configurée pour envoyer la commande d'activation (14) pour démarrer l'enregistrement des données et la commande de désactivation (15) à la fin de l'enregistrement des données aux unités électroniques (9).

8. Système selon l'une quelconque des revendications 6 ou 7, **caractérisé en ce que** l'application (18) est configurée pour effacer les données enregistrées dans les sections de mémoire (10) des unités électroniques (9).

9. Système selon l'une quelconque des revendications 6 à 8, **caractérisé en ce que** le dispositif électronique (17) est équipé d'un système de géolocalisation (19) et l'application (18) est configurée pour associer les données géographiques du système de géolocalisation (19) aux données enregistrées dans les sections de mémoire (10) des unités électroniques (9).

10. Système selon l'une quelconque des revendications 6 à 9, **caractérisé en ce que** l'application (18) est configurée pour envoyer les données enregistrées dans les sections de mémoire (10) à une plate-forme Web (20).

11. Système selon la revendication 10, **caractérisé en ce que** la plate-forme Web (20) est configurée pour recevoir des données de l'extérieur ainsi que de l'application (18).

12. Système selon la revendication 10 ou 11, **caractérisé en ce que** la plate-forme Web (20) est configurée pour traiter les données reçues de l'application (18) et pour mettre à la disposition d'un utilisateur les données traitées dans un format numérique et/ou graphique associé à une session motrice et est configurée pour extrapoler un ou plusieurs rapports d'une session motrice dérivés des analyses et des statistiques des données traitées.

13. Procédé de fonctionnement d'un ensemble d'activités motrices (1) selon l'une quelconque des revendications 1 à 5, comprenant une étape consistant à activer l'enregistrement des données acquises,
**caractérisé en ce que**
cette étape est associée à une étape consistant à envoyer un horodatage à au moins l'une des unités électroniques (9) du premier bâton (2) et du second bâton (3) ;
après l'étape consistant à activer l'enregistrement, une étape consistant à envoyer un horodatage à au moins l'une des unités électroniques (9) du premier bâton (2) et du second bâton (3) selon une fréquence de transmission prédéterminée.

14. Procédé selon la revendication 13, **caractérisé en ce qu'**il stocke les données acquises par les capteurs (8) associés à un horodatage relatif.
